# EUROPEAN PATENT SPECIFICATION

(11) **EP 3 522 860 B1**
(45) Date of publication and mention of the grant of the patent: **18.11.2020**
(21) Application number: 17771771.7
(22) Date of filing: 25.09.2017
(51) Int. Cl.: A61K 8/44, A61K 8/49, A61Q 5/00, A61Q 5/12

(54) **HAIR TREATMENT COMPOSITION**
HAARBEHANDLUNGSZUSAMMENSETZUNG
COMPOSITION DE TRAITEMENT CAPILLAIRE

(30) Priority: 05.10.2016 EP 16192482
(43) Date of publication of application: 14.08.2019
(73) Proprietor: Unilever PLC, London, Greater London EC4Y 0DY (GB); Unilever N.V., 3013 AL Rotterdam (NL)
(72) Inventor: BELL, Fraser, Ian, Bebington Wirral Merseyside CH63 3JW (GB); RICKETTS, Stephen, Robert, Leicester Leicestershire LE2 6EX (GB); ROBERTS, Glyn, Bebington Wirral Merseyside CH63 3JW (GB); TAYLOR, Cheryl, Anne, Bebington Wirral Merseyside CH63 3JW (GB)
(74) Representative: Chisem, Janet
(86) International application number: PCT/EP2017/074177
(87) International publication number: WO 2018/065248

(56) References cited:
- EP-A1- 1 142 904
- CH-B1- 697 229
- CN-A- 101 692 996
- FR-A1- 2 853 531
- FR-A1- 2 939 198
- US-A- 4 906 460
- DATABASE GNPD [Online] MINTEL; September 2016 (2016-09), "Keratin & Bifida Protein Hair Treatment", XP002764026, Database accession no. 4287193
- DATABASE GNPD [Online] MINTEL; March 2016 (2016-03), "Vegetal Keratin Restorative Mask", XP002764027, Database accession no. 3875661
- Lubrizol: "Restructuring Treatment for Damaged Hair", , 13 June 2012 (2012-06-13), XP002764028, Retrieved from the Internet: URL:https://www.lubrizol.com/PersonalCare/ Formulations/CD-0028(LA)-Restructuring-Tre atment-for-Damaged-Hair.html [retrieved on 2016-11-09]
- VILLA ET AL.: "Feather keratin hydrolysates obtained from microbial keratinases: Effect on hair fiber", BMC BIOTECHNOLOGY, vol. 13(1), no. 15, 2013, - 2013, pages 1-11, XP002764029, DOI: 10.1186/1472-6750-13-15
- OSHIMURA E. ET AL.: "Hair and amino acids: The interactions and the effects", J.COSMET.SCI., vol. 58, no. 4, July 2007 (2007-07), - August 2007 (2007-08), pages 347-357, XP002764030, ISSN: 1525-7886

## Description

### Field of invention

The present invention relates to a treatment composition for hair, comprising mixtures of amino acids, its use to mitigate the effect of chemical assaults, and a method of treating chemically damaged hair using said composition.

### Background and prior art

Hair is composed mainly of proteins, specifically keratins. Healthy hair has healthy proteins, whilst damaged hair has damaged proteins, depending on the extent and type of damage. Loss of protein and protein substituents, such as amino acids, is characteristic of damaged hair. The denaturation temperature of hair is a reliable indicator of the level of damage and loss of its constituent proteins. The damage to hair protein is reflected in a reduction in the denaturation temperature of the proteins. Once hair is damaged, it is more susceptible to further loss of proteins and therefore to further reduction of hair integrity and to further damage.

Hair is subjected to a range of "assaults" during everyday life or as part of a normal hair care regime or consumer habit. These include chemical assaults (for example, bleaching treatments, colouring treatments and use of surfactants). These assaults damage the hair structure and proteins.

This damage is manifested in numerous ways, including surface damage, chipped scales, lifted scales, loss of shine, loss of smoothness, hair breakage, split ends, cuticle damage and loss, and difficultly in styling. In addition, damaged hair is more porous, which means that it is weaker, more prone to breakage, frizzy fly-away and more susceptible to further damage. There is a need to mitigate these effects.

Treatments are known which address the problem of hair damage due to chemical hair treatments, and which may involve the use of amino acids.

EP 2 886 162A **(Henkel)** discloses a hair treatment agent containing a homopolymer or copolymer (comprising a vegetable oil monomer), a quaternary ammonium compound and an ester oil. It can also contain at least one amino acid. The composition aims to improve physical damage caused by grooming and blow-drying, and particularly to improve split ends or hair breakage. Damage caused by repeated hair treatments, such as oxidative or reductive hair treatments, as well as the too frequent cleansing of the hair, the action of heat during drying and environmental factors and/or mechanical effects, which can hair defraud in their structure, is also referred to.

WO 14/202655 **(L'Oreal)** discloses a composition for treating keratin fibres comprising a combination of an acrylic polymer particles, a silicone block copolymer and at least one amino acid or amino acid derivative. The composition effectively coats the hair and is persistent with respect to shampooing and to the various attacking factors to which the hair may be subjected, especially blow-drying and perspiration, while at the same time showing better tolerance towards fatty substances such as sebum without developing any tacky nature.

US 2015/272860 **(Henkel)** discloses a hair treatment composition including at least one amino acid and/or at least one oligopeptide and/or at least one cationic protein hydrolysate and a sugar structure-containing silicone. The composition aims to reduce the side effects of environmental influences and of oxidative and surfactant hair treatments, preferably during the oxidative and surfactant hair treatments.

WO 14/090513 **(Henkel)** discloses a composition for treating keratin fibres, without causing dryness and split ends, comprising quaternary ammonium compound, odoriferous substance, a zwitterionic or amphoteric surfactant and a silicone containing sugar structure. The compositions can include amino acids.

WO 12/031069 **(Zotos Int Inc)** discloses a treatment composition comprising a mixture of peptides made of any of Serine, Tyrosine, Arginine, Threanine, Glycine, Valine, Phenalanine, Cysteine and Lucine in the sequences identical to human hair keratin proteins; the composition aims to benefit hair strength, manageability and overall conditioning and can be substantive to hair using natural amino acids.

US 2008/058400 **(Fujifilm)** discloses a skin preparation that comprises at least arginine, aspartic acid, isoleucine, leucine, lysine, threonine, glycine, histidine, serine, valine, tyrosine, cysteine, phenylalanine, hydroxyproline and acylglutamine among amino acids, or salts thereof. It can be used as a face lotion, an emulsion, a cream, a hair tonic or a pack.

FR 2 853 531A **(Sephytal)** discloses an after-shampoo composition containing a mixture of free amino-acids identical with those in human hair and a quaternary amine salt with a 22C fatty chain of the behenyl type, derived from natural colza oil. The amino acids comprise glutamic acid, arginine, proline, aspartic acid, leucine, phenylalanine, serine , lysine, glycine, valine, tyrosine, isoleucine, alanine, threonine, histidine, methionine and cystine. The composition aims to confer to dry hair very dry, damaged or embrittled hair, a better ease of disentangling after washing and rinsing the hair, a gentle significantly improved breathability and more shine.

WO 00/51556 **(P&G)** discloses a hair care composition comprising four or more amino acids where each amino acid is selected from a different group of amino acids. The composition aims to treat hair that is subjected to a wide range of insults that can cause weakening and damage.

There remains a need for more effective treatments for damaged hair, which deliver repair benefits during every day hair-care regimes.

We have now found that a mixture of specific amino acids, namely glutamic acid, alanine and proline, can repair damage to hair that has been chemically damaged, as evidenced by an increase in the denaturation temperature of the hair. We have surprisingly found that the amino acids mixture of the invention act unusually quickly to repair hair damage by increasing the denaturation temperature of the hair. The compositions of the invention provide repair benefits after just one treatment and can return the denaturation temperature of 8 times bleached hair to that of virgin hair after only 3 treatments.

### Specific description of the invention

In a first aspect of the invention there is provided a hair rinse-off treatment composition as disclosed in present claim 1 comprising a mixture of amino acids, wherein the mixture of amino acids comprises glutamic acid, alanine and proline.

In a second aspect of the invention there is provided a method of treating chemically damaged hair as disclosed in present claim 3 comprising the step of applying to the hair a treatment composition as defined in the first aspect of the invention.

In a third aspect there is provided a use of a composition as defined in the first aspect to repair chemically damaged hair.

### General description

### The composition

### The mixture of amino acids

The amino acid mixture for use the compositions of the invention comprises, glutamic acid, alanine and proline. The mixture is preferably free from other amino acids.

The amino acid mixture is preferably used in solution or emulsified form. It may be dissolved or dispersed in a suitable solvent or carrier.

The composition of the invention is preferably an aqueous solution, The amount of the amino acid mixture in a hair composition is from 0.1 wt % to 10 wt %, more preferably from 0.2 wt % to 5 wt %, most preferably from 0.25 wt % to 2 wt %, by total weight of the composition.

The weight ratio of glutamic acid : alanine: proline is 2:1:1 to 1:2:1 to 1:1:2, more preferably 1:1:1.

Amino acids, including glutamic acid, alanine and proline, suitable for use in the invention are available from many suppliers, for example Kusuma Pharma and Ajinomoto co Inc.

### The Method

The method of the invention is a method of treating chemically damaged hair comprising the step of applying to the hair a hair treatment composition comprising a mixture of amino acids, wherein the mixture of amino acids comprises glutamic acid, alanine and proline.

The chemically damaged hair is preferably selected from hair that has been subjected to treatments that modify the hair, preferably bleaching treatments, colouring treatments, straightening treatments, relaxing treatments, surfactant treatments and hot water, more preferably bleaching treatments, colouring treatments, surfactant treatments and hot water, most preferably bleaching treatments.

Examples of surfactant treatments include shampoos.

Hair is often subjected to hot water during washing, treatment and rinsing processes. Typically, the temperature of the hot water is from 20 to 45 °C. Protein and amino acid leaching from hair increases with the temperature of the water.

Preferably, the hair has been subjected to multiple damaging treatments, preferably at least 2, more preferably at least 4 damaging treatments. For example, 8 bleach treatments.

The method comprises the step of applying to the hair a composition of the invention.

The method preferably comprises applying the composition of the invention to the hair multiple times, to give a progressive damage repair as shown by an increase in the denaturation temperature of the protein. In this way, it is possible to increase the denaturation temperature of the protein to equal to or even higher than that of virgin hair.

In the context of this invention, by virgin hair is meant hair that has not been subjected to intensive physical and/or chemical treatment, for example, bleaching, dyeing, perming, heat treatment and strong and/or prolonged exposure to solar radiation; nor displays features characteristic of damaged hair, for example, split ends and/or excessive dryness. Virgin hair includes hair that has sustained low levels of damage during the natural hair life cycle. Sources of low level damage likely include but are not necessarily limited to brushing, combing and natural processes such as limited solar photo-degradation, for example.

The method may thus additionally comprise the step of repeating the application of the composition to the hair, preferably during a later treatment. Preferably the step of repeating the application of the composition to the hair during a later treatment is repeated 1 to 10 times, more preferably from 1 to 5 times, most preferably from 1 to 3 times.

The composition of the present invention is a rinse-off composition.

For the purposes of the present invention, a rinse-off composition is a composition that is applied to the hair as part of a hair care process, and removed, by rinsing, preferably 1 second to 1 hour following application, more preferably after 20 seconds to 3 minutes, most preferably after 1 minute.

Rinse off compositions are selected from a shampoo, a conditioner and a treatment such as a mask or an oil, most preferably selected from a shampoo and a conditioner.

Preferably, consecutive treatment compositions, for example a shampoo and a conditioner, each comprising the amino acid mixture of the invention, are used one after the other. In one embodiment, the conditioner is applied to the hair before the shampoo, in a "reverse wash" process.

The method of the invention preferably comprises the steps of :
a) applying to the hair a composition comprising a mixture of amino acids comprising glutamic acid, alanine and proline; and
b) leaving the composition on the hair for 2 seconds to 20 minutes, more preferably 10 seconds to 3 minutes, most preferably 20 seconds to 1 minute.

The method comprises the step of removing the composition, preferably by rinsing.

### The Use

The invention provides a use of the composition of the invention to repair chemically damaged hair

The use of the present invention provides long lasting damage repair, preferably an increase in the denaturation temperature of protein. By long lasting means that the benefit lasts for multiple treatments, preferably from 2 to 5 treatments, with a hair composition that does not comprise the amino acid mixture of the invention.

### Method of making

Each amino acid can be added separately at different stages, during the manufacture of the compositions of the invention, or the same stage, for example as a premix. Alternatively, 2 or 3 of the amino acids can be premixed before addition. They can be added, for example, as a dispersion in water or combined with the fragrance oil.

### The hair treatment composition

Hair treatment compositions of the invention are primarily intended for topical application to the hair and/or scalp of a human subject, in rinse-off compositions, for the treatment of dry, damaged and/or unmanageable hair.

The composition of the invention comprises a surfactant.

Preferably, the compositions of the invention have a pH of from 3 to 7, more preferably from 3 to 6. Where the composition is a shampoo, the most preferred pH is from 4.5 to 5.

### Boiler plate

### Shampoos

Shampoo compositions of the invention are generally aqueous, i.e. they have water or an aqueous solution or a lyotropic liquid crystalline phase as their major component.

Suitably, the shampoo composition will comprise from 50 to 98%, preferably from 60 to 90% water by weight based on the total weight of the composition.

Shampoo compositions according to the invention will generally comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates,alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic cleansing surfactants are sodium lauryl sulphate, sodium lauryl ether sulphate (n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20).

Mixtures of any of the foregoing anionic cleansing surfactants may also be suitable.

The total amount of anionic cleansing surfactant in shampoo compositions of the invention generally ranges from 0.5 to 45%, preferably from 1.5 to 35%, more preferably from 5 to 20% by total weight anionic cleansing surfactant based on the total weight of the composition.

Optionally, a shampoo composition of the invention may contain further ingredients as described below to enhance performance and/or consumer acceptability.

The composition can include co-surfactants, to help impart aesthetic, physical or cleansing properties to the composition.

An example of a co-surfactant is a nonionic surfactant, which can be included in an amount ranging from 0.5 to 8%, preferably from 2 to 5% by weight based on the total weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO-(G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 8%, preferably from 1 to 4% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine. A suitable example is Tegobetaine CK, ex Evonik. Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a shampoo composition of the invention is generally from 1 to 50%, preferably from 2 to 40%, more preferably from 10 to 25% by total weight surfactant based on the total weight of the composition.

Cationic polymers are preferred ingredients in a shampoo composition of the invention for enhancing conditioning performance.

Suitable cationic polymers may be homopolymers which are cationically substituted or may be formed from two or more types of monomers. The weight average (M_{w}) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quatemization.

The cationic polymers can comprise mixtures of monomer units derived from amine- and/or quaternary ammonium-substituted monomer and/or compatible spacer monomers.
Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.
Cationic polysaccharide polymers suitable for use in compositions of the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15 and JAGUAR C17.

Mixtures of any of the above cationic polymers may be used.

Cationic polymer will generally be present in a shampoo composition of the invention at levels of from 0.01 to 5%, preferably from 0.05 to 1%, more preferably from 0.08 to 0.5% by total weight of cationic polymer based on the total weight of the composition.

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition.

Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10%, preferably from 0.5 to 6%, more preferably from 0.9 to 4% by total weight of suspending agent based on the total weight of the composition.

### Conditioners

Conditioner compositions will typically comprise one or more cationic conditioning surfactants which are cosmetically acceptable and suitable for topical application to the hair.

Preferably, the cationic conditioning surfactants have the formula N⁺(R¹)(R²)(R³)(R⁴), wherein R¹, R², R³ and R⁴ are independently (C₁ to C₃₀) alkyl or benzyl.

Preferably, one, two or three of R¹, R², R³ and R⁴ are independently (C₄ to C₃₀) alkyl and the other R¹, R², R³ and R⁴ group or groups are (C₁-C₆) alkyl or benzyl.

More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups. Suitable cationic conditioning surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (e.g., Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quatemium-5, Quatemium-31 and Quatemium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic conditioning surfactants for use in the invention, either alone or in admixture with one or more other cationic conditioning surfactants, is a combination of (i) and (ii) below:
an amidoamine corresponding to the general formula (I): 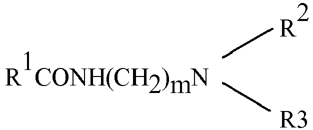
   in which R¹ is a hydrocarbyl chain having 10 or more carbon atoms,
   R² and R³ are independently selected from hydrocarbyl chains of from 1 to 10 carbon atoms, and
   m is an integer from 1 to about 10; and
an acid.
As used herein, the term hydrocarbyl chain means an alkyl or alkenyl chain.
Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and
m is an integer from 1 to about 4.
Preferably, R² and R³ are methyl or ethyl groups.
Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachid-amidoethyldiethylamine, arachidamidoethyldimethylamine, and mixtures thereof. Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate all the amidoamine present, i.e. at a level which is at least equimolar to the amount of amidoamine present in the composition.
In conditioners of the invention, the level of cationic conditioning surfactant will generally range from 0.01 to 10%, more preferably 0.05 to 7.5%, most preferably 0.1 to 5% by total weight of cationic conditioning surfactant based on the total weight of the composition.

Conditioners of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.01 to 10%, preferably from 0.1 to 8%, more preferably from 0.2 to 7%, most preferably from 0.3 to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Hair treatment compositions according to the invention, particularly water-based shampoos and hair conditioners, will preferably also contain one or more silicone conditioning agents.

Particularly preferred silicone conditioning agents are silicone emulsions such as those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvem Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC2-8177 and DC939 (from Dow Coming) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula:

HO(CH₂CH₂O)ₓ(CH(CH₃)CH₂O)_{y}(CH₂CH₂O)ₓH

wherein the mean value of x is 4 or more and the mean value of y is 25 or more.

Another preferred form of the surface active block copolymer is according to the following formula:

(HO(CH₂CH₂O)ₐ(CH(CH₃)CH₂O)_{b})₂-N-CH₂-CH₂-N((OCH₂CH(CH₃))_{b}(OCH₂CH₂)ₐOH)₂

wherein the mean value of a is 2 or more and the mean value of b is 6 or more.

Mixtures of any of the above described silicone emulsions may also be used.
The above described silicone emulsions will generally be present in a composition of the invention at levels of from 0.05 to 10%, preferably 0.05 to 5%, more preferably from 0.5 to 2% by total weight of silicone based on the total weight of the composition.

A preferred conditioner comprises a conditioning gel phase. Such conditioners and methods for making them are described in WO2014/016354, WO2014/016353, WO2012/016352 and WO2014/016351.

### Other Ingredients

A composition of the invention may contain other ingredients for enhancing performance and/or consumer acceptability. Such ingredients include fragrance, dyes and pigments, pH adjusting agents, pearlescers or opacifiers, viscosity modifiers, and preservatives or antimicrobials. Each of these ingredients will be present in an amount effective to accomplish its purpose. Generally these optional ingredients are included individually at a level of up to 5% by weight of the total composition.

Hair treatment compositions of the invention are primarily intended for topical application to the hair and/or scalp of a human subject, either in rinse-off or leave-on compositions, for the treatment of dry, damaged and/or unmanageable hair.

The invention will be further illustrated by the following, non-limiting Examples, in which all percentages quoted are by weight based on total weight unless otherwise stated.

### EXAMPLES

### Example 1 - Preparation of shampoo compositions. Shampoo 1 & Shampoo 2, in accordance with the invention and of comparative shampoos. Shampoo A & Shampoo B.

Shampoos 1 and 2, in accordance with the invention contained a 1:1:1 wt mixture of the amino acids glutamic acid, alanine and proline. Shampoos A and B were the same as Shampoos 1 and 2, only without the amino acids.

All the shampoos were prepared using the following method:
1. In the compositions in accordance with the invention, the amino acids (glutamic acid, alanine and proline) were added to water in a suitable vessel, with stirring.
2. Any Carbopol was then added.
3. SLES and CAPB were then added.
4. The mixture was heated to 30° C and mixed until completely homogenous.
5. Any guar polymer was then added and mixed well.
6. Fragrance was then added.
7. Silicone conditioning agent was added, if using, and mixed.
8. Hydrolysed protein was added, if using.
9. The pearliser was then added.
10. The preservatives were added.
11. The pH was adjusted to pH 4 to 5 using a mixture of 50% sodium hydroxide/50% citric acid.
12. Salt and polypropylene glycol were then added to adjust the viscosity as desired.

Shampoo 1 and Shampoo A were prepared with a viscosity in the range of from 9,000 to 15,000 cps (as measured by a Brookfield viscosmeter, rv 5 spindle at 20 rpm for 1 minute at 30 °C).

Shampoo 2 and Shampoo B were prepared with a viscosity in the range of from 5,000 to 9,000 cps (as measured by a Brookfield viscosmeter, rv 5 spindle at 20 rpm for 1 minute at 30 °C).

The compositions of Shampoo 1, Shampoo 2, Shampoo A and Shampoo B are shown in Table 1 below:

**Table 1: Compositions of Shampoos 1 & 2 and Shampoos A & B**

| | **Amount (wt % of 100 % active)** | | | |
|---|---|---|---|---|
| **Ingredient** | **Shampoo 1** | **Shampoo A** | **Shampoo 2** | **Shampoo B** |
| Carbopol 980 | - | - | 10.0 | 10.0 |
| Sodium Lauryl Ether Sulphate (SLES) + cocamidopropyl betaine (CAPB) | 13.5 | 13.5 | 22.0 | 22.0 |
| Guar polymer | - | - | 0.2 | 0.2 |
| Pearliser | 1.2 | 1.2 | 2.0 | 2.0 |
| Silicone conditioning agent* | - | - | 1.7 | 1.7 |
| Fragrance | 0.8 | 0.8 | 0.8 | 0.8 |
| Hydrolysed Protein | 2.3 | 2.3 | - | - |
| Glutamic Acid | 0.67 | - | 0.67 | - |
| Alanine | 0.67 | - | 0.67 | - |
| Proline | 0.67 | - | 0.67 | - |
| Preservative | 0.55 | 0.55 | 0.55 | 0.55 |
| Sodium hydroxide | To pH | to pH | pH | to pH |
| Citric acid soln | To pH | to pH | pH | to pH |
| Polypropylene glycol (PPG) and salt | To viscosity | To viscosity | To viscosity | To viscosity |
| Water | To 100 | To 100 | To 100 | To 100 |

| | | | | |
|---|---|---|---|---|
| *Dimethicone, ex Dow Corning | | | | |

### Example 2 - Preparation of conditioner compositions. Conditioner 1, Conditioner 2 and Conditioner 3 in accordance with the invention and of comparative conditioners. Conditioner A, Conditioner B and Conditioner C.

Conditioners 1, 2 and 3 in accordance with the invention contained a 1:1:1 wt mixture of the amino acids glutamic acid, alanine and proline. Conditioners A, B and C were the same as Conditioners 1, 2 and 3 respectively, only without the amino acids.

The conditioners were prepared using the following method:
1. Water was added to a suitable vessel and heated to 80 °C.
2. Cetearyl alcohol was then added to the formulation along with tertiary amine salt (TAS) (if present in the formulation).
3. The formulation was allowed to cool to 80°C the Behenyl Trimethyl Ammonium Chloride (BTAC) was added and the resultant mixture mixed until opaque and thick.
4. If TAS was present in the formulation, lactic acid was added and the formulation was stirred for a further 10 minutes.
5. The heat was then turned off and the quench water was added.
6. The amino acids were added.
7. The mixture was then cooled to below 55°C and the EDTA and NaCl were added, as a predissolved pre mix.
8. Once the formulation had cooled to below 40°C the rest of the materials, including fragrance, were added.
9. Finally the formulation was mixed at high shear on a Silverson mixer at 5000rpm for 5 minutes.

The compositions of Conditioners 1, 2 and 3, and Comparative Conditioners A, B and C are shown in Table 2 below:

**Table 2: Compositions of Conditioners 1, 2 and 3, and Comparative Conditioners A, B and C**

| | **Amount (wt % of 100 % active)** | | | | | |
|---|---|---|---|---|---|---|
| **Ingredient** | **Conditioner 1** | **Conditioner A** | **Conditioner 2** | **Conditioner B** | **Conditioner 3** | **Conditioner C** |
| Quaternary surfactant* | 1.25 | 1.25 | 1.0 | 1.0 | 2.3 | 2.3 |
| TAS** | 1.25 | 1.25 | 1.0 | 1.0 | - | - |
| FATTY ALC | 5.0 | 5.0 | 4.0 | 4.0 | 3.2 | 3.2 |
| Conditioning silicone*** | 1.4 | 1.4 | 2.14 | 2.14 | 1.4 | 1.4 |
| Lactic acid | 1.1 | 1.1 | 0.85 | 0.85 | - | - |
| Preservative | 0.5 | 0.5 | 0.5 | 0.5 | 0.5 | 0.5 |
| Glutamic acid | 0.33 | - | 0.66 | - | 0.33 | - |
| Alanine | 0.33 | - | 0.66 | - | 0.33 | - |
| Proline | 0.33 | - | 0.66 | - | 0.33 | - |
| Fragrance | 1.0 | 1.0 | 1.0 | 1.0 | 1.0 | 1.0 |
| Water | To 100 | To 100 | To 100 | To 100 | To 100 | To 100 |

| | | | | | | |
|---|---|---|---|---|---|---|
| *Genamin BTLF, ex Clariant **Lexamine s13, ex Inolex ***Dimethicone, ex Dow Corning | | | | | | |

### Example 3 - Preparation of a gel composition. Gel 1, in accordance with the invention and of a comparative gel, Gel A.

Gel 1, in accordance with the invention contained a 1:1:1 wt mixture of the amino acids glutamic acid, alanine and proline. Comparative Gel A was the same as Gel 1, only without the amino acids.

The gels were prepared using the following method:
The ingredients were added to a suitable vessel, from highest percentage inclusion to the lowest. The final addition was the thickening polymer. The resultant mixture was stirred at 30°C.

The compositions of Gel 1 and Gel A are shown in Table 3 below.

**Table 3: Compositions of Gel 1 and Gel A**

| | **Amount (wt % of 100 % active)** | |
|---|---|---|
| **Ingredient** | **Gel 1** | **Gel A** |
| Propylene Glycol | 5.0 | 5.0 |
| Hydrolysed Collagen | 11.0 | 11.0 |
| Hydrolyzed Elastin | 3.0 | 3.0 |
| Polyacrylamide polymer Thickener | 40.0 | 40.0 |
| Preservative | 0.2 | 0.2 |
| Hydrolysed Keratin 20 | 1.2 | 1.2 |
| L-Glutamic acid | 0.34 | - |
| Alanine | 0.33 | - |
| Proline | 0.33 | - |
| Water | To 100 | To 100 |

### Example 4: Measurement of the denaturation temeratures and change in denaturation temperature of hair, using Differential Scanning Calorimetry (DSC), to give an indication of the level of damage in hair treated with Shampoo 1 & Shampoo 2, in accordance with the invention and of comparative shampoos. Shampoo A & Shampoo B Conditioner 1, Conditioner 2 and Conditioner 3 in accordance with the invention and of comparative conditioners. Conditioner A, Conditioner B and Conditioner C

The internal protein of damaged hair typically has a reduced denaturation temperature compared to that of virgin hair. Damage repair is evidenced by an increase in the denaturation temperature of the internal protein of hair.

### The Hair

### Virgin:

The hair used in the following examples was dark brown European hair tresses 5 grams and 10 inches long.

### Double-bleached:

Virgin hair tresses were bleached according to the following protocol. Hair was bleached twice for 30 minutes with Platine Precision White Compact Lightening Powder (L'Oreal Professionnel Paris, Paris, France) mixed with 9% cream peroxide, 30 'vol' (Excel GS Ltd, UK) (60 g of powder mixed with 120 g cream peroxide). Hair was then washed with 14% SLES solution after the second treatment before drying. "

### Treatment of the Hair

Hair was given a preliminary wash as follows:
Hair was immersed in water for 30 s before being immersed in 14 % aq sles solution. The hair was rubbed for 30 s, rinsed for 30 s and then treated with a non-conditioning shampoo, using 0.1 ml/1g hair, with rubbing for 30 s and rinsing for 30 s.

The hair (virgin and double bleached) was then treated with the compositions as detailed in Examples 1 and 2 above, using the following method:
The shampoo composition was applied to hair in an amount of 0.1 ml/1g hair and rubbed for 30 seconds to lather, followed by 30 seconds rinse in tap water.

The conditioner composition was applied to hair in an amount of 0.2ml/1 g hair and rubbed for 60 seconds, followed by a 60 seconds rinse in tap water.

The hair tresses were then left to dry overnight at 20°C at 50 % relative humidity.

The effect of the treatments was measured using Differential Scanning Calorimetry (DSC).
The bottom 2 cm of the treated hair switches was removed using scissors. The hair was then chopped small using hair clippers. 7-10 mg of the clippings were added to the aluminium pans of the Calorimeter, along with 50µL of water. In all cases 5 hair switches were used per treatment and 1 pan was prepared per hair switch. The prepared pans were allowed to sit overnight before being run on the DSC from 100-180°C, at a rate of 5°C/min.

**Table 5: Mean denaturation temperatures for double bleached hair treated with Compositions Shampoo 1 & Shampoo 2, in accordance with the invention and of comparative shampoos. Shampoo A & Shampoo B Conditioner 1, Conditioner 2 and Conditioner 3 in accordance with the invention and of comparative conditioners. Conditioner A, Conditioner B and Conditioner C**

| **Treatment** | **Denaturation Temperature** | **Standard Deviation** | **Statistical Grouping** |
|---|---|---|---|
| | | | **Tukey Kramer (p<0.05)** |
| **Conditioner A** | 144.417 | 0.249065 | D,E |
| 1 wash | | | |
| **Conditioner A** | 145.018 | 0.632844 | C,D |
| 5 washes | | | |
| **Conditioner 1** | 143.51 | 0.683959 | E |
| 1 wash | | | |
| **Conditioner 1** | 146.498 | 0.27127 | B,C |
| 5 washes | | | |
| **Conditioner B** | 143.68 | 1.125033 | D,E |
| 1 wash | | | |
| **Conditioner B** | 146.1467 | 0.748151 | B,C |
| 5 washes | | | |
| **Conditioner 2** | 150.42 | 0.572974 | |
| 1 wash | | | |
| **Conditioner 2** | 148.786 | 0.748151 | A |
| 5 washes | | | |
| **Conditioner C** | 143.68 | 1.125033 | D,E |
| 1 wash | | | |
| **Conditioner C** | 146.1467 | 0.748151 | B,C |
| 5 washes | | | |
| **Conditioner 3** | 147.9 | 0.127279 | A,B |
| 1 wash | | | |
| **Conditioner 3** | 147.0767 | 0.291433 | B |
| 5 washes | | | |
| Double bleached (control) | 144.41 | 0.27074 | D,E |
| Virgin (control) | 146.423 | 0.28219 | B,C |

It will be seen that damage repair, shown by an increase in the denaturation temperature, occurs with treatment by the shampoo compositions in accordance with the invention.

**Shampoos**

| **Treatment** | **Denaturation Temperature** | **Standard Deviation** | **Statistical Grouping Tukey Kramer (p<0.05)** |
|---|---|---|---|
| **Shampoo A** | 146.567 | 0.506195 | C |
| 1 Wash | | | |
| **Shampoo A** | 148.17 | 0.5781 | B |
| 5 washes | | | |
| **Shampoo 1** | 147.864 | 0.872829 | B |
| 1 wash | | | |
| **Shampoo 1** | 149.615 | 0.279583 | A |
| 5 washes | | | |
| **Shampoo B** | 145.9325 | 0.179141 | C |
| 1 wash | | | |
| **Shampoo B** | 146.0925 | 0.236132 | C |
| 5 washes | | | |
| **Shampoo 2** | 146.74 | 0.422137 | C |
| 1 wash | | | |
| **Shampoo 2** | 150.01 | 0.141421 | A |
| 5 washes | | | |
| Double bleached (control) | 144.41 | 0.27074 | D |
| Virgin (control) | 146.423 | 0.28219 | C |

It will be seen that damage repair, shown by an increase in the denaturation temperature, occurs with treatment by the conditioner compositions in accordance with the invention.

## Claims

1. A hair rince off treatment composition comprising a mixture of amino acids, wherein the mixture of amino acids comprises glutamic acid, alanine and proline, **characterised in that** the weight ratio of glutamic acid: alanine: proline is 2:1:1 to 1:2:1 to 1:1:2 and the mixture of amino acids is 0.1 to 10 % by weight of the total composition, and wherein the composition is a shampoo or a conditioner and comprises a surfactant.

2. A composition as claimed in claim 1, wherein the pH is in the range of from 3 to 7.

3. A method of treating chemically damaged hair comprising the step of applying to the hair a treatment composition as defined in any one of claims 1 to 2.

4. A method as claimed in claim 3 wherein the chemically damaged is damaged by bleaching treatments, colouring treatments, straightening treatments, relaxing treatments, surfactant treatments, hot water from 20 to 45°C and mixtures thereof.

5. A method as claimed in claim 4, which additionally comprises the step of leaving the composition on the hair for 2 seconds to 20 minutes, preferably 10 seconds to 3 minutes, most preferably 20 seconds to 1 minute.

6. A method as claimed in any one claims 3 to 5, which additionally comprises the step of repeating the application of the composition to the hair.

7. A method as claimed in claim 6, wherein the step of repeating the application of the composition to the hair is made during a later treatment and is repeated 1 to 10 times.

8. A use of a composition as defined in any one of claims 1 to 2 to repair chemically damaged hair.

9. A use of a composition as defined in any one of claims 1 to 2 to provide long lasting damage repair, such that the benefit lasts for multiple treatments, preferably from 2 to 5 treatments, with a hair composition that does not comprise the amino acid mixture of the invention.

## Patentansprüche

1. Rinse-off-Haarbehandlungszusammensetzung, umfassend eine Mischung von Aminosäuren, wobei die Mischung von Aminosäuren Glutaminsäure, Alanin und Prolin umfasst, **dadurch gekennzeichnet, dass** das Gewichtsverhältnis von Glutaminsäure : Alanin : Prolin 2:1:1 bis 1:2:1 bis 1:1:2 beträgt und die Mischung von Aminosäuren 0,1 bis 10 Gewichts-% der Gesamtzusammensetzung beträgt, und wobei die Zusammensetzung ein Shampoo oder eine Haarspülung ist und ein Tensid umfasst.

2. Zusammensetzung wie in Anspruch 1 beansprucht, wobei der pH im Bereich von 3 bis 7 liegt.

3. Verfahren zur Behandlung von chemisch geschädigtem Haar, umfassend den Schritt des Aufbringens einer Behandlungszusammensetzung, wie in irgendeinem der Ansprüche 1 bis 2 definiert, auf das Haar.

4. Verfahren wie in Anspruch 3 beansprucht, wobei das chemisch Geschädigte durch Bleichbehandlungen, Färbebehandlungen, Glätterbehandlungen, Glättungsbehandlungen, Tensidbehandlungen, heißes Wasser von 20 bis 45 °C und Mischungen davon beschädigt wird.

5. Verfahren wie in Anspruch 4 beansprucht, das zusätzlich den Schritt umfasst, die Zusammensetzung auf dem Haar für 2 Sekunden bis 20 Minuten, bevorzugt 10 Sekunden bis 3 Minuten, am meisten bevorzugt 20 Sekunden bis 1 Minute, zu lassen.

6. Verfahren wie in irgendeinem der Ansprüche 3 bis 5 beansprucht, das zusätzlich den Schritt des Wiederholens des Aufbringens der Zusammensetzung auf das Haar umfasst.

7. Verfahren wie in Anspruch 6 beansprucht, wobei der Schritt des Wiederholens des Aufbringens der Zusammensetzung auf das Haar während einer späteren Behandlung erfolgt und 1 bis 10 Mal wiederholt wird.

8. Verwendung einer Zusammensetzung wie in irgendeinem der Ansprüche 1 bis 2 definiert, um chemisch geschädigtes Haar zu reparieren.

9. Verwendung einer Zusammensetzung wie in irgendeinem der Ansprüche 1 bis 2 definiert, um eine langandauernde Schadensreparatur bereitzustellen, so dass der Nutzen für mehrere Behandlungen, bevorzugt 2 bis 5 Behandlungen, mit einer Haarzusammensetzung, die die Aminosäuremischung der Erfindung nicht umfasst, andauert.

## Revendications

1. Composition de traitement à rincer pour les cheveux comprenant un mélange d'acides aminés, dans laquelle le mélange d'acides aminés comprend de l'acide glutamique, de l'alanine et de la proline, **caractérisée en ce que** le rapport massique d'acide glutamique : alanine : proline est de 2:1:1 à 1:2:1 à 1:1:2 et le mélange d'acides aminés est de 0,1 à 10 % en masse de la composition totale, et dans laquelle la composition est un shampoing ou un après-shampoing et comprend un tensioactif.

2. Composition selon la revendication 1, dans laquelle le pH se trouve dans l'intervalle de 3 à 7.

3. Procédé de traitement de cheveux chimiquement endommagés comprenant l'étape d'application aux cheveux d'une composition de traitement selon l'une quelconque des revendications 1 à 2.

4. Procédé selon la revendication 3, dans lequel le dommage chimique est un dommage par des traitements de décoloration, des traitements de coloration, des traitements de lissage, des traitements relaxants, des traitements de tensioactifs, l'eau chaude de 20 à 45°C et des mélanges de ceux-ci.

5. Procédé selon la revendication 4, qui comprend de plus l'étape de laisser la composition sur les cheveux pendant de 2 secondes à 20 minutes, de préférence de 10 secondes à 3 minutes, encore mieux de 20 secondes à 1 minute.

6. Procédé selon l'une quelconque des revendications 3 à 5, qui comprend de plus l'étape de répétition de l'application de la composition aux cheveux.

7. Procédé selon la revendication 6, dans lequel l'étape de répétition de l'application de la composition aux cheveux est réalisée pendant un traitement ultérieur et est répétée de 1 à 10 fois.

8. Utilisation d'une composition selon l'une quelconque des revendications 1 à 2 pour réparer des cheveux chimiquement endommagés.

9. Utilisation d'une composition selon l'une quelconque des revendications 1 à 2 pour fournir une réparation de longue durée du dommage, de sorte que le bénéfice dure pendant de multiples traitements, de préférence de 2 à 5 traitements, avec une composition de cheveux qui ne comprend pas le mélange d'acides aminés de l'invention.
